# EUROPEAN PATENT APPLICATION

(11) **EP 2 530 464 A1**
(43) Date of publication of application: **05.12.2012**
(21) Application number: 10844563.6
(22) Date of filing: 27.12.2010
(51) Int. Cl.: G01N 33/53, G01N 33/48

(54) **AMYLOID MEASUREMENT METHOD**

(30) Priority: 28.01.2010 JP 2010016559
(71) Applicant: Panasonic Corporation, Kadoma-shi Osaka 571-8501 (JP)
(72) Inventor: NANJOH, Toshifumi, Osaka 540-6207 (JP); FUKUHARA, Takaomi, Osaka 540-6207 (JP)
(74) Representative: TBK
(86) International application number: PCT/JP2010/007546
(87) International publication number: WO 2011/092796

(57) **Abstract**

Disclosed herein is an Aβ measurement method capable of accurately measuring the concentration of Aβ in a sample even when the sample contains a very low level (about several pM) of Aβ. The amyloid β measurement method includes: a sample preparation step 101 in which a sample possibly containing amyloid β is placed in a sample treatment vessel; a concentration step 102 in which a solubilizer that solubilizes amyloid β is added to the sample in the sample treatment vessel and the amount of solvent contained in the sample is reduced; a neutralization step 103 in which the solubilizer in a treated sample solution obtained in the concentration step is neutralized; and a measurement step 104 in which amyloid β possibly contained in a neutralized treated sample solution is quantitatively measured based on an antigen-antibody reaction.

## Description

### TECHNICAL FIELD

The present invention relates to a highly-sensitive amyloid β (hereinafter, also referred to as "Aβ") measurement method.

### BACKGROUND ART

Alzheimer's disease is a disease characterized by progressive dementia that occurs in elderly and senile age. Now, it is said that the number of domestic patients with the disease is 1,000,000 or more. It is conceivable that the number will surely increase in the future as the population ages. Examples of the clinical symptoms of Alzheimer's disease are dysmnesia and higher brain dysfunction (aphasia, apraxia, agnosia, and constructive apraxia). Such symptoms are often observed also in other demential diseases, and therefore it is very difficult to definitely diagnose Alzheimer's disease by clinical symptoms alone.

There has been no basic remedy for Alzheimer's disease. However, since vaccine therapy in model mice succeeded in 1999, expectations for development of basic remedies have increased (see Non-Patent Document 1). In order to effectively use such basic remedies, it is necessary to early diagnose Alzheimer's disease.

Histopathological findings characteristic of Alzheimer's disease include senile plaques and neurofibrillary tangles in brain tissue. The former are mainly composed of Aβ aggregates having a β sheet structure and the latter are mainly composed of hyperphosphorylated tau proteins. At present, amyloid hypothesis that an initial pathological change occurring at the onset of Alzheimer's disease is accumulation of Aβ is dominant (see Non-Patent Document 2). That is, it is known that, in the case of Alzheimer's disease, the above-described pathological tissue change such as accumulation of Aβ in the brain progresses before the onset of clinical symptoms. Therefore, detection of Aβ peptides in the brain as markers is one of early diagnosis methods of diseases caused by accumulation of Aβ, especially Alzheimer's disease.

As in-vitro diagnostic agents for Alzheimer's disease, those designed based on the principle of ELISA using antibodies specific to Aβ are widely used. It has become virtually certain by ELISA that Aβ constituted from 42 amino acids (Aβ42) in cerebrospinal fluid decreases with progression of Alzheimer's disease (see Non-Patent Document 3).

### RELATED ART DOCUMENTS

### Non-Patent Documents

Non-Patent Document 1: Schenk D, Seubert P et al., Nature 400: 173-177, 1999
Non-Patent Document 2: Hardy JA, Selkose DF, Science 297: 353-356, 2002
Non-Patent Document 3: Hansson O, Mithon L et al., Lancet Neurol 5: 228-234, 2006

### SUMMARY OF THE INVENTION

### Problem to be Solved by the Invention

However, when a sample containing a very low level (e.g., about several pM) of Aβ, such as an irrigation solution obtained by irrigation of living tissue, is subjected to quantitative measurement based on an antigen-antibody reaction such as the above-described ELISA, Aβ cannot be accurately measured due to its very low concentration.

In order to solve the above problem, it is an object of the present invention to provide an Aβ measurement method capable of accurately measuring the concentration of Aβ in a sample even when the concentration of Aβ in the sample is as very low as about several pM.

### Means for Solving the Problem

In order to achieve the above object, the present invention is directed to an amyloid β measurement method including: a sample preparation step in which a sample possibly containing amyloid β is placed in a sample treatment vessel; a concentration step in which a solubilizer that solubilizes amyloid β is added to the sample in the sample treatment vessel and the amount of solvent contained in the sample is reduced by a concentration operation; a neutralization step in which the solubilizer in a treated sample solution obtained in the concentration step is neutralized; and a measurement step in which amyloid β possibly contained in the neutralized treated sample solution is quantitatively measured based on an antigen-antibody reaction.

The present invention is also directed to an amyloid β measurement method including: a sample treatment vessel preparation step in which an additive to be attached to amyloid β is placed in a sample treatment vessel; a sample preparation step in which a sample possibly containing amyloid β is placed in the sample treatment vessel; a first concentration step in which the sample in the sample treatment vessel is concentrated; a second concentration step in which a solubilizer that solubilizes amyloid β is added to the sample concentrated in the first concentration step and the amount of solvent contained in the sample is reduced by a concentration operation; a neutralization step in which the solubilizer in a treated sample solution obtained in the second concentration step is neutralized; and a measurement step in which amyloid β possibly contained in a neutralized treated sample solution is quantitatively measured based on an antigen-antibody reaction.

### Effect of the Invention

The amyloid β measurement method according to the present invention makes it possible to accurately measure the concentration of Aβ in a sample even when the sample contains a very low level (e.g., about several pM) of Aβ.

### BRIEF DESCRIPTION OF THE DRAWINGS

Fig. 1 is a flow chart showing the steps of Example 1.
Fig. 2 is a graph showing the results of Aβ concentration measurement in Example 1.
Fig. 3 is a flow chart showing the steps of Example 2.
Fig. 4 is a graph showing the results of Aβ concentration measurement in Example 2.
Fig. 5 is a flow chart showing the steps of Example 3.
Fig. 6 is a graph showing the results of Aβ recovery rate measurement in Example 3.
Fig. 7 is a flow chart showing the steps of Example 4.
Fig. 8 is a graph showing the results of Aβ recovery rate measurement in Example 4.
Fig. 9 is a flow chart showing the steps of Reference Example.
Fig. 10 is a graph showing the results of Aβ recovery rate measurement in Reference Example.
Fig. 11 is a flow chart showing the steps of an Aβ measurement method according to a first embodiment of the present invention.
Fig. 12 is a flow chart showing the steps of an Aβ measurement method according to a second embodiment of the present invention.
Fig. 13 is a graph showing the difference in Aβ recovery rate between the presence or absence of a solubilizer.

### DESCRIPTION OF EMBODIMENTS

Aβ to be measured by an Aβ measurement method according to the present invention is a highly-aggregative and highly-adsorptive material. This is because many of amino acids constituting Aβ are highly hydrophobic.

The phrase "highly-aggregative" means that Aβ is likely to self-associate and aggregate. Here, Aβ that is present as a free molecule without forming an aggregate is referred to as an Aβ monomer. An aggregate of two or more Aβ molecules, which is soluble in an aqueous solution such as a normal saline solution, is referred to as a soluble Aβ multimer or a soluble Aβ oligomer (hereinafter, collectively referred to as a soluble Aβ oligomer). Aβ that is insoluble in the above-described aqueous solution due to the progress of aggregation from the above-described soluble Aβ oligomer state is referred to as an insoluble Aβ multimer or an insoluble Aβ polymer (hereinafter, collectively referred to as an insoluble Aβ polymer). The reason why Aβ is highly aggregative is that highly-hydrophobic Aβ is usually more stable in the form of an aggregate when present in an aqueous solution.

The phrase "highly-adsorptive" means that Aβ is likely to be adsorbed to materials other than Aβ. An example of the materials other than Aβ is the surface of a vessel. Another example of the materials other than Aβ is living tissue. In either case, hydrophobic interaction between the hydrophobic region of the material other than Aβ and the hydrophobic region of Aβ is considered as one of the causes.

Particularly important Aβ monomers are an Aβ monomer composed of 42 amino acids (hereinafter, also referred to as "Aβ42") and an Aβ monomer composed of 40 amino acids (hereinafter, also referred to as "Aβ40"). It is believed that the formation of senile plaques in brain tissue, known as a hallmark of Alzheimer's disease, results from the above-described aggregative properties and adsorptive properties of Aβ42 and Aβ40. At present, Aβ42 and Aβ40 contained in spinal fluid are measurement indicators in actual clinical practice. Aβ42 is relatively more aggregative and adsorptive than Aβ40. In the present invention, "Aβ" is a general term for Aβ monomers, soluble Aβ oligomers, and insoluble Aβ polymers, unless otherwise specified. Further, "Aβ monomer" refers to Aβ42 and Aβ40, unless otherwise specified.

A sample used in the Aβ measurement method according to the present invention is a body fluid (e.g., cerebrospinal fluid, blood serum, tears, nasal secretion, and saliva) collected from a patient with Alzheimer's disease or a subject suspected of having Alzheimer's disease. The sample may be nasal secretion or nasal mucosal scrapings obtained by collecting nasal mucus from the nasal mucosa of the above-described subject with the use of a collection tool such as a cotton swab or a swab. The sample may be an irrigation solution obtained by irrigation of living tissue (e.g., brain tissue or mucosa), in or to which the above-described Aβ is possibly aggregated or adsorbed, with a normal saline solution or the like.

In the present invention, nasal secretion or nasal mucosal scrapings obtained by collecting nasal mucus from the nasal mucosa of the above-described subject with the use of a collection tool such as a cotton swab or a swab can be used as a sample. This is because Aβ in the brain tends to be accumulated in tissue around the nasal mucosa. More specifically, nasal mucus can be collected using a swab or a cotton swab (e.g., a γ-sterilized aluminum shaft cotton swab manufactured by Eiken Chemical Co., Ltd.). The sample may be nasal mucus itself collected by rubbing a swab or a cotton swab against the nasal mucosa or an extract obtained by extracting nasal mucus from the cotton swab with a predetermined extraction liquid. Here, examples of the predetermined extraction liquid include normal saline solutions, surfactant-containing solutions, and organic solvents.

In the present invention, particularly, an irrigation solution obtained by irrigation of the nasal mucosa with a normal saline solution or the like can be used as a sample. This is because Aβ in the brain tends to be accumulated in tissue around the nasal mucosa. More specifically, the above-described irrigation solution can be collected by using a commercially-available nasal irrigator. For example, a normal saline solution is injected into one of the nostrils using a nasal irrigator manufactured and marketed by Izumi products company (product name: INC-7000, INC-7200, INC-7001), and then the normal saline solution discharged from the other nostril after irrigation is collected. The total amount of the solution obtained by nasal irrigation in this way can be used as a sample in the present invention. It is to be noted that the irrigation solution to be injected into the nasal cavity is not limited to a normal saline solution as long as it is a solution having biocompatibility.

The amount of Aβ contained in a sample to be measured by the Aβ measurement method according to the present invention may be very small. Aβ is strongly aggregated in or adsorbed to living tissue such as mucosa, and therefore in the above-described case where nasal mucus is collected by a collection tool such as a swab or a cotton swab, the collection tool needs to be strongly rubbed against the nasal mucosa repeatedly. However, the amount of Aβ contained in the thus obtained nasal mucus is very small, and therefore when the nasal mucus is extracted with an extraction liquid, the concentration of Aβ in a sample becomes lower. Further, in the above-described case where nasal irrigation is performed, it is difficult to collect Aβ when the amount of a normal saline solution is small, and therefore nasal irrigation needs to be performed using a large volume of normal saline solution. Therefore, the concentration of Aβ in an obtained sample is very low.

Most of Aβ contained in living tissue such as mucosa are soluble Aβ oligomers or insoluble Aβ polymers, and the amount of Aβ monomers is very small. Therefore, when nasal mucus and an irrigation solution obtained by nasal irrigation were directly measured by ELISA using, for example, Aβ monomer assay kits manufactured and marketed by Wako Pure Chemical Industries Ltd., the presence of Aβ could not be detected. Next, a sample whose Aβ40 and Aβ42 concentrations were adjusted to achieve predetermined concentrations (Aβ40: 50 pM, Aβ42: 10 pM) at the time of measurement was prepared, and the sample was concentrated under a reduced pressure and measured by ELISA. As a result, the presence of Aβ was very slightly detected. From the result, it is considered that the concentrations of Aβ40 monomer and Aβ42 monomer in nasal mucus and an irrigation solution obtained by nasal irrigation are as very low as less than 50 pM and less than 10 pM, respectively.

Aβ is accumulated mainly in brain tissue, but a very small amount of Aβ is secreted also into other somatic cells or body fluids such as blood, spinal fluid, and nasal secretion. However, when a sample is collected from, for example, the nasal mucosa, a swab or a cotton swab is rubbed against the nasal mucosa or the nasal mucosa is irrigated with an irrigation solution. When a sample is collected using a swab or a cotton swab, collected nasal mucus is preferably extracted into an extraction liquid. When a sample is collected by nasal irrigation, the nasal mucosa is preferably irrigated with a large amount of irrigation solution. In either case, the concentration of Aβ in the sample is reduced. Therefore, the concentration of Aβ in the sample needs to be increased by subjecting the sample to a concentration operation before measurement.

The concentration operation can be usually performed by concentration under a reduced pressure. The present inventors have focused attention on a concentration step, and have done experiments repeatedly under different conditions or by different methods. As a result of intensive studies, it has been found that, in a concentration step, the amount of solvent contained in a sample is reduced by vaporization of the solvent, but in the course of this step, Aβ40 monomers or Aβ42 monomers contained in the sample aggregate together so that aggregates thereof are produced in large amounts. That is, it has been found that when a sample is directly subjected to a concentration step, Aβ40 monomers or Aβ42 monomers contained in the sample aggregate together to form tight aggregates so that the amount of Aβ40 monomer or Aβ42 monomer contained in the sample is significantly reduced and as a result the value of Aβ measured by ELISA is reduced.

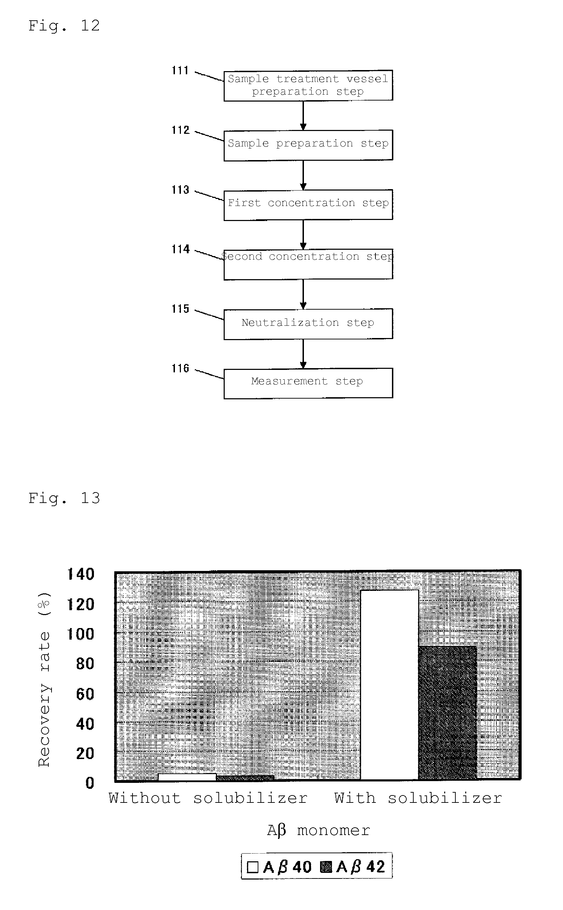

The present inventors have found that the measurement sensitivity of Aβ is significantly improved by preventing the aggregation of monomers with each other in a concentration step by adding a solubilizer such as formic acid to a sample before the sample is subjected to the concentration step. The results are shown in Fig. 13. A sample was prepared by adjusting the concentrations of the above-described standard substances to predetermined values. When the sample was concentrated after formic acid was added thereto, the measured value of Aβ was about 26 to 28 times larger than that when the sample was concentrated without adding formic acid. From the results, it has been found that when a solubilizer such as formic acid is added to a sample before the sample is subjected to a concentration step, very highly-sensitive measurement of Aβ can be performed as compared to when the sample is directly subjected to the concentration step without adding the solubilizer.

The Aβ measurement method according to the present invention includes: a sample preparation step in which a sample possibly containing amyloid β is placed in a sample treatment vessel; a concentration step in which a solubilizer that solubilizes amyloid β is added to the sample in the sample treatment vessel and the amount of solvent contained in the sample is reduced by a concentration operation; a neutralization step in which the solubilizer in a treated sample solution obtained in the concentration step is neutralized; and a measurement step in which amyloid β possibly contained in a neutralized treated sample solution is quantitatively measured based on an antigen-antibody reaction. The method according to the present invention includes the above steps, and therefore Aβ contained in a sample is solubilized before measurement and Aβ monomers formed by solubilization are kept even after the concentration step, which makes it possible to quantitatively measure Aβ more accurately.

In the Aβ measurement method according to the present invention, an additive to be attached to Aβ is preferably placed in a sample treatment vessel before a sample is placed in the sample treatment vessel. This makes it possible to inhibit the aggregation of Aβ due to the additive attached to Aβ.

Further, in the present invention, when a large volume of sample such as an irrigation solution obtained by nasal irrigation is measured, performing a pre-concentration step before the above-described concentration step is effective. More specifically, the method according to the present invention preferably includes, before the concentration step (a second concentration step), a pre-concentration step (a first concentration step) in which the sample in the sample treatment vessel is concentrated. This makes it possible to efficiently perform the next concentration step (the second concentration step).

Further, the method according to the present invention preferably includes a sample treatment vessel preparation step in which an additive to be attached to Aβ is previously placed in a sample treatment vessel to be used in the above-described sample preparation step. This makes it possible to increase the recovery rate of Aβ and therefore to quantitatively measure Aβ more accurately. Particularly, when the above-described pre-concentration step is performed, the progress of insolubilization of Aβ in the pre-concentration step can be prevented by previously placing an additive to be attached to Aβ in a sample treatment vessel, which makes it possible to quantitatively measure Aβ more accurately.

The Aβ measurement method according to the present invention includes: a sample treatment vessel preparation step in which an additive to be attached to amyloid β is placed in a sample treatment vessel; a sample preparation step in which a sample possibly containing amyloid β is placed in the sample treatment vessel; a first concentration step in which the sample in the sample treatment vessel is concentrated; a second concentration step in which a solubilizer that solubilizes amyloid β is added to the sample concentrated in the first concentration step and the amount of solvent contained in the sample is reduced by a concentration operation; a neutralization step in which the solubilizer in a treated sample solution obtained in the second concentration step is neutralized; and a measurement step in which amyloid β possibly contained in the neutralized treated sample solution is quantitatively measured based on an antigen-antibody reaction.

### First Embodiment

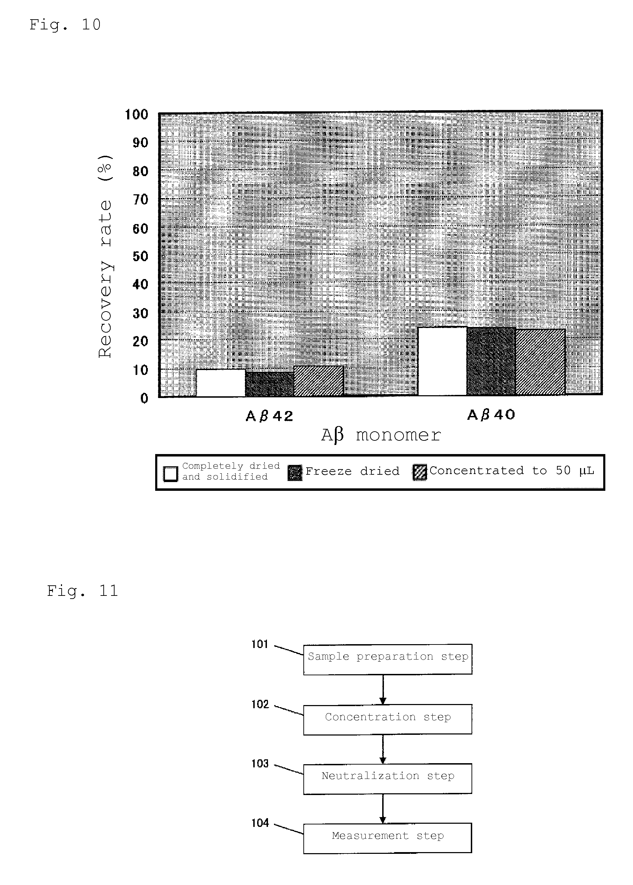

Hereinbelow, each of the steps of a first embodiment of the Aβ measurement method according to the present invention will be described in detail with reference to Fig. 11.

In a sample preparation step 101 of the Aβ measurement method according to the present invention, a sample possibly containing Aβ is placed in a sample treatment vessel.

The sample treatment vessel used in the Aβ measurement method according to the present invention preferably has an inner wall surface having the property of inhibiting adsorption of Aβ to inhibit adsorption of Aβ to the inner wall surface of the vessel to increase the recovery rate of Aβ. More specifically, the vessel preferably has an inner wall surface that is not hydrophobic. For example, a glass vessel is generally preferred. Alternatively, a vessel having an inner wall surface treated with silicone or the like to be hydrophilic may be used. The use of such a vessel makes it possible to reduce hydrophobic interaction between highly-hydrophobic Aβ monomers and the inner wall of the vessel, thereby inhibiting adsorption of Aβ monomers to the inner wall surface of the vessel. This makes it possible to increase the recovery rate of Aβ.

The use of a blocking agent makes it possible to further inhibit adsorption of Aβ monomers to the inner wall of the vessel. "Blocking agent" is a generic name for reagents containing a protein or a compound that is not reacted with or bound to Aβ monomers. Specific examples of the blocking agent include bovine serum albumin (BSA), skimmed milk, casein, and surfactants. The interaction between such a blocking agent and the inner wall surface of the vessel makes it possible to relatively inhibit adsorption of Aβ present in the sample in low concentration to the inner wall surface of the vessel. The blocking agent is usually used in a concentration of 0.01%w/v to 5%w/v. When viscosity or the like is a concern, the blocking agent is used in a concentration of 0.01%w/v to 0.5%w/v.

When the blocking agent is used, the blocking agent needs to be present in the vessel when the sample is placed in the vessel. More specifically, the sample is charged into the vessel in which a solution containing the blocking agent is liquid-tightly enclosed. Alternatively, the sample in the form of liquid may be charged into the vessel in which the blocking agent kept in a dry form is placed so as to be resoluble. Alternatively, the blocking agent may be adsorbed and fixed to the inner wall surface of the vessel by previously bringing a solution containing the blocking agent into contact with the inner wall surface of the vessel. Alternatively, the blocking agent or a solution containing the blocking agent may be added after the sample is added to the vessel. It is to be noted that the blocking agent is usually dissolved in a buffer solution. Examples of the buffer solution include phosphate buffers and Tris buffers. The use of the blocking agent is preferred because the recovery rate of Aβ further increases, but may be omitted.

The capacity and shape of the sample treatment vessel used in the present invention are not particularly limited. The capacity of the sample treatment vessel may be arbitrarily set as long as the vessel can hold the sample. Examples of the vessel having a capacity of 1 mL to 2 mL include Eppendorf tubes, examples of the vessel having a capacity of 50 mL or less include Corning tubes, and examples of the vessel having a capacity of 1000 mL or less include beakers and flasks. The shape of the sample treatment vessel may also be arbitrarily selected depending on the method of subsequent treatment. Here, the treatment method is a method usually used in biochemical experiments or examinations, such as centrifugal separation. It is often the case that the above-mentioned Eppendorf tubes and Corning tubes are shaped to fit into the rotor of a centrifugal separator frequently used.

Then, a concentration step 102 is performed, in which a solubilizer that solubilizes Aβ is added to the sample in the sample treatment vessel to obtain a mixture and the mixture is subjected to a concentration operation to reduce the amount of solvent contained in the sample. In the concentration step 102, Aβ oligomers possibly contained in the sample are treated with the solubilizer to convert them into Aβ monomers. By converting Aβ in the sample into Aβ monomers by treatment using the solubilizer, Aβ can be quantitatively measured more accurately because Aβ measurement based on an antibody-antigen reaction is highly sensitive to Aβ monomers. Further, by reducing the amount of solvent contained in the sample, it is possible to increase the concentration of Aβ in the sample and therefore to achieve more sensitive measurement.

As the solubilizer, formic acid may be used. Formic acid is usually used to solubilize proteins aggregated in or adsorbed to animal or human tissue to separate them from the tissue. However, the present inventors have found that soluble Aβ oligomers dispersed in a liquid containing no living tissue are dissociated into Aβ monomers by the action of formic acid on the soluble Aβ oligomers. Formic acid to be used preferably has a concentration of 70% or higher when added to the sample. A mechanism for dissociation of soluble Aβ oligomers into Aβ monomers is unclear, but it is conceivable that a few of soluble oligomers are reversibly dissociated into Aβ monomers in a liquid and, in such a state, formic acid contributes to stabilization of Aβ monomers.

In the present invention, the solubilizer is not limited to formic acid. As the solubilizer, an organic acid having the ability to convert soluble Aβ oligomers into Aβ monomers can be used. Examples of such an organic acid include organic acids having a carboxyl group or a sulfo group. Specific examples of such an organic acid include, in addition to formic acid, acetic acid, oxalic acid, malic acid, citric acid, tartaric acid, trifluoroacetic acid, phthalic acid, fumaric acid, maleic acid, succinic acid, methanesulfonic acid, benzenesulfonic acid, and p-toluenesulfonic acid. These solubilizers may be used singly or in combination of two or more of them.

The solubilizer used in the concentration step 102 preferably contains, in addition to formic acid, a compound having a double bond, a triple bond, or a conjugate bond (e.g., S-allyl-L-cysteine (hereinafter, abbreviated as "SAC")). This makes it possible to further increase the recovery rate of Aβ.

In the concentration step 102, concentration may be performed by concentration under a reduced pressure. In the concentration step 102 of the Aβ measurement method according to the present invention, the sample is preferably concentrated without insolubilizing Aβ. The sample contains Aβ monomers or soluble oligomers. Insolubilization of Aβ refers to a phenomenon in which a solid phase composed of Aβ is deposited by formation of insoluble Aβ polymers due to the progress of aggregation of the above-described Aβ monomers or soluble oligomers during concentration. The phrase "the sample is concentrated without insolubilizing Aβ" in the present invention means that the sample is concentrated without being dried and solidified or a phenomenon, in which the amount of insoluble Aβ polymers (i.e., the amount of solid phase composed of Aβ) in the sample is substantially increased during concentration by concentrating the sample without neutralizing formic acid added as the solubilizer, does not occur. The concentration step 102 is performed to increase the concentration of Aβ in the sample, but the sample is concentrated without increasing the amount of insoluble Aβ polymers, that is, Aβ monomers obtained by adding the solubilizer are kept as they are, which makes it possible to quantitatively measure Aβ accurately.

In order to concentrate such a sample without drying and solidifying it as described above, the amount of solvent to be reduced in the concentration step needs to be adjusted. That is, if the amount of solvent to be reduced is too large, the sample is dried and solidified, and therefore the amount of solvent to be reduced is controlled. Here, the phrase "the sample is dried and solidified" means that the amount of solvent contained in the sample becomes relatively small so that the sample loses fluidity as a whole and is solidified. Therefore, in order to concentrate the sample without drying and solidifying it, the concentration step needs to be finished in a state where the liquid sample keeps its fluidity. If the sample is dried and solidified in the concentration step, as will be described later with reference to Example 3, the measurement sensitivity of Aβ is reduced due to aggregation of Aβ monomers or soluble oligomers. Therefore, in the concentration step of the method according to the first embodiment, it is preferred that the amount of solvent contained in the sample is reduced without drying and solidifying the sample. This makes it possible to significantly improve the measurement sensitivity of Aβ.

Then, a neutralization step 103 is performed, in which a neutralizer is added to a treated sample solution obtained in the concentration step 102 to neutralize the solubilizer contained in the treated sample solution. When an organic acid such as formic acid is used as the solubilizer in the concentration step 102, the treated sample solution is highly acidic, and therefore neutralization needs to be performed for a subsequent measurement step 104.

For example, formic acid can be neutralized by adding 1M Tris as the neutralizer in a predetermined amount.

Then, the treated sample solution neutralized in the neutralization step 103 is subjected to the measurement step 104 to quantitatively measure Aβ based on an antigen-antibody reaction. The measurement step 103 of the Aβ measurement method according to the present invention utilizes an immunoassay based on an antigen-antibody reaction. The sample such as an irrigation solution obtained by nasal irrigation contains not only Aβ as a measuring object but also many proteins and foreign matter. In order to measure only Aβ that is a measuring object contained in the sample, a substance that specifically reacts with Aβ is preferably used. For this reason, an immunoassay is utilized. An immunoassay is an assay method utilizing an antigen-antibody reaction as an immunoreaction, and is widely used as a measuring principle because an antibody against a substance to be measured can be artificially produced. Actually, Aβ assay kits are marketed by Wako Pure Chemical Industries. However, when the sample contains a low level of Aβ, Aβ cannot be quantitatively measured accurately simply by using such commercially-available Aβ assay kits. According to the present invention, even when the sample contains a very low level of Aβ, Aβ can be quantitatively measured accurately by performing the above-described concentration step in the presence of the solubilizer.

The measurement step 104 is intended to perform quantitative measurement, and therefore, in the concentration step 102, the amount of concentrate (the amount of liquid remaining after concentration) is preferably controlled to be constant. The amount of concentrate can be easily controlled by adding a high-boiling medium that does not form an azeotrope with a water-based medium, such as dimethylsulfoxide (DMSO), to the treated sample solution. In this case, when the amount of the high-boiling medium to be added is adjusted to a constant value, only the water-based medium is vaporized by concentration utilizing water vaporization and the high-boiling medium such as DMSO remains, and therefore a constant amount of concentrate can be stably obtained.

Alternatively, the amount of concentrate may be controlled by optical measurement. More specifically, a predetermined position of the vessel is irradiated with light, and in the course of concentration, concentration is stopped when a light scatter signal generated by the movement of meniscus of the treated sample solution to the predetermined position is detected.

Alternatively, the amount of concentrate may be controlled by electrochemically detecting the amount of liquid remaining. More specifically, an electrode pair is provided at a predetermined position of the vessel, and in the course of concentration, concentration is stopped when the flow of an electric current is stopped by the passage of meniscus of the treated sample solution through the electrode pair provided at the predetermined position.

Alternatively, the amount of concentrate may be controlled by providing a means for measuring the volume of vaporized liquid and calculating the amount of liquid remaining. Alternatively, the amount of concentrate may be controlled by providing a means for obtaining a constant amount of concentrate by utilizing the weight of concentrate. For example, a piezoelectric element is attached to the vessel to measure the amount of mass change.

The above-described measurement method according to the first embodiment including all the steps 101 to 104 is particularly effective when nasal mucus collected from the nasal mucosa with a collection tool such as a cotton swab or a swab is used as a sample. This is because when nasal mucus is used as a sample, the volume of the sample is small, and therefore the sample can be concentrated after the solubilizer is directly added to the sample.

### Second Embodiment

Hereinbelow, each of the steps of a second embodiment of the Aβ measurement method according to the present invention will be described in detail with reference to Fig. 12.

In a sample treatment vessel preparation step 111 of the Aβ measurement method according to the present invention, an additive to be attached to Aβ is placed in a sample treatment vessel. The present inventors have found that aggregation of Aβ monomers and soluble oligomers with each other can be inhibited by the action of the additive on the Aβ monomers and the soluble Aβ oligomers. An example of the additive is formic acid. The concentration of formic acid to be used may be less than 70% because the additive does not need to have the ability to convert soluble Aβ oligomers into Aβ monomers.

In the present invention, the additive is not limited to formic acid. As the additive, an organic acid having the ability to inhibit aggregation of Aβ monomers and soluble oligomers with each other can be used. Examples of such an organic acid include organic acids having a carboxyl group or a sulfo group. Specific examples of such an organic acid include, in addition to formic acid, acetic acid, oxalic acid, malic acid, citric acid, tartaric acid, trifluoroacetic acid, phthalic acid, fumaric acid, maleic acid, succinic acid, methanesulfonic acid, benzenesulfonic acid, and p-toluenesulfonic acid. These additives may be used singly or in combination of two or more of them.

The additive used in the sample treatment vessel preparation step 111 preferably contains, in addition to formic acid, a compound having a double bond, a triple bond, or a conjugate bond (e.g., S-allyl-L-cysteine (hereinafter, abbreviated as "SAC")). This makes it possible to further increase the recovery rate of Aβ.

The sample treatment vessel preparation step 111 is intended to place an additive in a sample treatment vessel, which makes it possible to increase the recovery rate of Aβ and therefore to quantitatively measure Aβ more accurately. However, previously placing an additive in a sample treatment vessel is not essential to the present invention. For example, an additive may be added after a sample preparation step 112 is performed using a sample treatment vessel containing no additive.

The sample treatment vessel used in the Aβ measurement method according to the present invention preferably has an inner wall surface having the property of inhibiting adsorption of Aβ to inhibit adsorption of Aβ to the inner wall surface of the vessel to increase the recovery rate of Aβ. More specifically, the vessel preferably has an inner wall surface that is not hydrophobic. For example, a glass vessel is generally preferred. Alternatively, a vessel having an inner wall surface treated with silicone or the like to be hydrophilic may be used. The use of such a vessel makes it possible to reduce hydrophobic interaction between highly-hydrophobic Aβ monomers and the inner wall of the vessel, thereby inhibiting adsorption of Aβ monomers to the inner wall surface of the vessel. This makes it possible to increase the recovery rate of Aβ.

The use of a blocking agent makes it possible to further inhibit adsorption of Aβ monomers to the inner wall of the vessel. "Blocking agent" is a generic name for reagents containing a protein or a compound that is not reacted with or bound to Aβ monomers. Specific examples of the blocking agent include bovine serum albumin (BSA), skimmed milk, casein, and surfactants. The interaction between such a blocking agent and the inner wall surface of the vessel makes it possible to relatively inhibit adsorption of Aβ, present in a sample in low concentration, to the inner wall surface of the vessel. The blocking agent is usually used in a concentration of 0.01%w/v to 5%w/v. When viscosity or the like is a concern, the blocking agent is used in a concentration of 0.01%w/v to 0.5%w/v.

When the blocking agent is used, the blocking agent needs to be present in the vessel when a sample is placed in the vessel. More specifically, a sample is charged into the vessel in which a solution containing the blocking agent is liquid-tightly enclosed. Alternatively, a sample in the form of liquid may be charged into the vessel in which the blocking agent kept in a dry form is placed so as to be resoluble. Alternatively, the blocking agent may be adsorbed and fixed to the inner wall surface of the vessel by previously bringing a solution containing the blocking agent into contact with the inner wall surface of the vessel. Alternatively, the blocking agent or a solution containing the blocking agent may be added after a sample is added to the vessel. It is to be noted that the blocking agent is usually dissolved in a buffer solution. Examples of the buffer solution include phosphate buffers and Tris buffers. The use of the blocking agent is preferred because the recovery rate of Aβ further increases, but may be omitted.

The capacity and shape of the sample treatment vessel used in the present invention are not particularly limited. The capacity of the sample treatment vessel may be arbitrarily set as long as the vessel can hold the sample. Examples of the vessel having a capacity of 1 mL to 2 mL include Eppendorf tubes, examples of the vessel having a capacity of 50 mL or less include Corning tubes, and examples of the vessel having a capacity of 1000 mL or less include beakers and flasks. The shape of the sample treatment vessel may also be arbitrarily selected depending on the method of subsequent treatment. Here, the treatment method is a method usually used in biochemical experiments or examinations, such as centrifugal separation. It is often the case that the above-mentioned Eppendorf tubes and Corning tubes are shaped to fit into the rotor of a centrifugal separator frequently used.

Then, a sample preparation step 112 is performed, in which a sample is placed in the prepared sample treatment vessel.

Then, a first concentration step 113 is performed, in which the sample in the sample treatment vessel is concentrated. By performing the first concentration step 103, it is possible to increase the concentration of Aβ in the sample. This makes it easy to measure Aβ later and makes it possible to efficiently perform subsequent solubilization. When the first concentration step 113 is performed, the sample treatment vessel preparation step 111 is preferably performed in which an additive for solubilizing Aβ is previously placed in a sample treatment vessel. This makes it possible to prevent the progress of insolubilization of Aβ in the first concentration step 113. In the first concentration step 113, as will be described later with reference to Reference Example, the sample is concentrated without being dried and solidified.

Then, a second concentration step 114 is performed, in which a solubilizer that solubilizes Aβ is added to the sample treatment vessel containing the sample concentrated in the first concentration step to obtain a mixture, and the mixture is subjected to the second concentration operation to reduce the amount of solvent contained in the sample. In the second concentration step 114, Aβ oligomers possibly contained in the sample are treated with the solubilizer to convert them into Aβ monomers. By converting Aβ in the sample into Aβ monomers by treatment using the solubilizer, Aβ can be quantitatively measured more accurately because Aβ measurement based on an antibody-antigen reaction is highly sensitive to Aβ monomers. Further, by reducing the amount of solvent contained in the sample, it is possible to increase the concentration of Aβ in the sample and therefore to achieve more sensitive measurement.

As the solubilizer, formic acid may be used. Formic acid is usually used to solubilize proteins aggregated in or adsorbed to animal or human tissue to separate them from the tissue. However, the present inventors have found that soluble Aβ oligomers dispersed in a liquid containing no living tissue are dissociated into Aβ monomers by the action of formic acid on the soluble Aβ oligomers. Formic acid to be used preferably has a concentration of 70% or higher when added to the sample. A mechanism for dissociation of soluble Aβ oligomers into Aβ monomers is unclear, but it is conceivable that a few of soluble oligomers are reversibly dissociated into Aβ monomers in a liquid and, in such a state, formic acid contributes to stabilization of Aβ monomers.

In the present invention, the solubilizer is not limited to formic acid. As the solubilizer, an organic acid having the ability to convert soluble Aβ oligomers into Aβ monomers can be used. Examples of such an organic acid include organic acids having a carboxyl group or a sulfo group. Specific examples of such an organic acid include, in addition to formic acid, acetic acid, oxalic acid, malic acid, citric acid, tartaric acid, trifluoroacetic acid, phthalic acid, fumaric acid, maleic acid, succinic acid, methanesulfonic acid, benzenesulfonic acid, and p-toluenesulfonic acid. These solubilizers may be used singly or in combination of two or more of them.

The solubilizer used in the second concentration step 114 preferably contains, in addition to formic acid, a compound having a double bond, a triple bond, or a conjugate bond (e.g., S-allyl-L-cysteine (hereinafter, abbreviated as "SAC")). This makes it possible to further increase the recovery rate of Aβ.

In the second concentration step 114, concentration may be performed by concentration under a reduced pressure. In the second concentration step 114 of the Aβ measurement method according to the present invention, the sample is preferably concentrated without insolubilizing Aβ. The sample contains Aβ monomers or soluble oligomers. Insolubilization of Aβ refers to a phenomenon in which a solid phase composed of Aβ is deposited by formation of insoluble Aβ polymers due to the progress of aggregation of the above-described Aβ monomers or soluble oligomers during concentration. The phrase "the sample is concentrated without insolubilizing Aβ" in the present invention means that the sample is concentrated without being dried and solidified or a phenomenon, in which the amount of insoluble Aβ polymers (i.e., the amount of solid phase composed of Aβ) in the sample is substantially increased during concentration by concentrating the sample without neutralizing formic acid added as the solubilizer, does not occur. The second concentration step 114 is performed to increase the concentration of Aβ in the sample, but the sample is concentrated without increasing the amount of insoluble Aβ polymers, that is, Aβ monomers obtained by adding the solubilizer are kept as they are, which makes it possible to quantitatively measure Aβ accurately.

A measurement step 116 subsequent to the second concentration step 114 is intended to perform quantitative measurement, and therefore, in the second concentration step 114, the amount of concentrate (the amount of liquid remaining after concentration) is preferably controlled to be constant. The amount of concentrate can be easily controlled by adding a high-boiling medium that does not form an azeotrope with a water-based medium, such as dimethylsulfoxide (DMSO), to a treated sample solution. In this case, when the amount of the high-boiling medium to be added is adjusted to a constant value, only the water-based medium is vaporized by concentration utilizing water vaporization and the high-boiling medium such as DMSO remains, and therefore a constant amount of concentrate can be stably obtained.

Alternatively, the amount of concentrate may be controlled by optical measurement. More specifically, a predetermined position of the vessel is irradiated with light, and in the course of concentration, concentration is stopped when a light scatter signal generated by the movement of meniscus of a treated sample solution to the predetermined position is detected.

Alternatively, the amount of concentrate may be controlled by electrochemically detecting the amount of liquid remaining. More specifically, an electrode pair is provided at a predetermined position of the vessel, and in the course of concentration, concentration is stopped when the flow of an electric current is stopped by the passage of meniscus of a treated sample solution through the electrode pair provided at the predetermined position.

Alternatively, the amount of concentrate may be controlled by providing a means for measuring the volume of vaporized liquid and calculating the amount of liquid remaining. Alternatively, the amount of concentrate may be controlled by providing a means for obtaining a constant amount of concentrate by utilizing the weight of concentrate. For example, a piezoelectric element is attached to the vessel to measure the amount of mass change.

When the sample is a salt-containing solution such as an irrigation solution obtained by nasal irrigation, there is a case where the concentration of salt in the sample is increased by concentration in the concentration step of the Aβ measurement method according to the present invention, which adversely affects subsequent measurement performed by, for example, an enzyme immunoassay. In this case, the present invention may be performed after the sample is desalinated using a column or ultrafiltration or the amount of liquid in the sample is previously reduced.

Then, a neutralization step 115 is performed, in which a neutralizer is added to a treated sample solution obtained in the second concentration step 114 to neutralize the solubilizer contained in the treated sample solution. When an organic acid such as formic acid is used as the solubilizer in the second concentration step 114, the treated sample solution is highly acidic, and therefore neutralization needs to be performed for a subsequent measurement step 116.

For example, formic acid can be neutralized by adding 1M Tris as the neutralizer in a predetermined amount.

Then, the treated sample solution neutralized in the neutralization step 115 is subjected to the measurement step 116 to quantitatively measure Aβ based on an antigen-antibody reaction. The measurement step 116 of the Aβ measurement method according to the present invention utilizes an immunoassay based on an antigen-antibody reaction. The sample such as an irrigation solution obtained by nasal irrigation contains not only Aβ as a measuring object but also many proteins and foreign matter. In order to measure only Aβ that is a measuring object contained in the sample, a substance that specifically reacts with Aβ is preferably used. For this reason, an immunoassay is utilized. An immunoassay is an assay method utilizing an antigen-antibody reaction as an immunoreaction, and is widely used as a measuring principle because an antibody against a substance to be measured can be artificially produced. Actually, Aβ assay kits are marketed by Wako Pure Chemical Industries. However, when the sample contains a low level of Aβ, Aβ cannot be quantitatively measured accurately simply by using such commercially-available Aβ assay kits. According to the present invention, even when the sample contains a very low level of Aβ, Aβ can be quantitatively measured accurately by performing the above-described concentration step in the presence of the solubilizer.

The above-described measurement method according to the second embodiment including all the steps 111 to 116 is particularly effective when the sample is one having a large volume and containing a very low level of Aβ, such as an irrigation solution obtained by nasal irrigation. This is because when the sample is an irrigation solution obtained by nasal irrigation, the volume of the sample is large, and therefore direct addition of the solubilizer to the sample further increases the amount of liquid. For this reason, the solubilizer is preferably added after the amount of liquid in the sample is reduced by performing the first concentration step.

Examples of the immunoassay for measuring Aβ of interest in the present invention include enzyme immunoassay methods, fluorescence immunoassay methods, chemiluminescent immunoassay methods, and electrochemiluminescent immunoassay methods. In these methods, an antigen as a measuring object and an antibody that specifically binds to the measuring object and is labeled with an enzyme, a fluorescent substance, a chemiluminescent reactive substance, or an electrochemiluminescent substance are reacted and bound to each other, and then the unbound labeled antibody is separated and removed. Then, an optical or electrochemical signal generated by the enzyme, fluorescent substance, chemiluminescent reactive substance or electrochemiluminescent substance used as a label of the labeled antibody bound to the antigen is detected. These immunoassay techniques require a process for separating an antigen-antibody complex of an antigen as a measuring object and an antibody that specifically binds to the measuring object from the unbound labeled antibody. Therefore, these immunoassay techniques are collectively referred to as an immunoassay with BF (which is an abbreviation for "bind-free") separation.

More specifically, the above-described assay kit manufactured by Wako Pure Chemical Industries is based on the principle of an enzyme immunoassay. This assay kit is composed of a microtiter plate on which a first antibody against Aβ monomer (Aβ40, Aβ42) is immobilized, a second Aβ antibody labeled with HRP, and an enzyme chromogenic substrate. The Aβ40/42 ELISA kit can detect 1 pM to 100 pM of Aβ40 and 0.1 pM to 20 pM of Aβ42. The time required for assay is 17 hours. The Aβ40/42 ELISA kit is highly sensitive. A measurement method using the Aβ40/42 ELISA kit is as follows. First, a sample liquid is dispensed into the microtiter plate, and then the microtiter plate is washed with a buffer solution, and then the second antibody is added. Then, the microtiter plate is washed with a buffer solution, and then the chromogenic substrate is added for color development to measure absorbency.

The immunoassay for measuring Aβ of interest in the present invention may be an immunoassay without BF separation. Examples of such an immunoassay without BF separation include turbidimetric immunoassay methods, nephelometric immunoassay methods, and latex turbidimetric immunoassay methods. In these methods, antigen-antibody aggregates generated by a binding reaction between an antigen as a measuring object and an antibody that specifically binds to the measuring object are optically measured. More specifically, a change in size caused by the antigen-antibody binding reaction is detected as an optical change. In the terbidimetric immunoassay methods or the latex terbidimetric immunoassay methods, a measuring object is quantitatively measured by detecting turbidity generated by an antigen-antibody reaction as the amount of change in the intensity of transmitted light. In the nephelometric immunoassay methods, a measuring object is quantitatively measured by detecting a change in the size of aggregates produced by an antigen-antibody reaction as the amount of change in the intensity of scattered light.

An antibody used in the immunoassay for measuring Aβ of interest in the present invention is one that specifically reacts with and binds to Aβ. Several methods for producing such an antibody have been established and are already well known. A typical production method is one in which a mouse is immunized with a measuring object as an antigen and then the spleen of the mouse is extracted to produce hybridoma cells (antibody-producing cells) by the fusion of the spleen and myeloma cells. What is important here is an antigen. In the case of the present invention, Aβ is an antigen. More specifically, Aβ42 and Aβ40 are antigens. Further, what is important is which region of Aβ is recognized by and bound to an antibody that binds to an antigen. For example, when differentiating between Aβ42 and Aβ40, an antibody needs to recognize a region whose amino acid sequence is different between Aβ42 and Aβ40. More specifically, Aβ42 and Aβ40 are different in the C-terminal amino acid sequence, and therefore an antibody that can recognize an amino acid sequence containing C-terminal two residues is specific to Aβ42 and an antibody that does not is specific to Aβ40. Aβ42 and Aβ40 have the same N-terminal amino acid sequence, and therefore an antibody that recognizes the N-terminal region binds to both Aβ42 and Aβ40. The above-described assay kit manufactured by Wako Pure Chemical Industries uses an appropriate combination of these antibodies.

As described above, the Aβ measurement method according to the present invention utilizes the fact that Aβ in the brain is accumulated in the nasal mucosa and tissue around the nasal mucosa with age, and therefore can use, as a sample, nasal mucus obtained by rubbing a swab or a cotton swab against the nasal mucosa or the like or an irrigation solution obtained by irrigation of the nasal mucosa or the like. In this case, the method according to the present invention has the advantage that it is useful in clinical researches and diagnosis because the amount of Aβ in the nasal cavity including the nasal bone and the nasal mucosa can be measured without taking a sample from the nasal bone. Further, the method according to the present invention has the advantage that highly-sensitive measurement of Aβ can be achieved by concentrating a sample without aggregation of Aβ, and therefore the amount of Aβ contained in a sample taken from the nose or the like, which cannot be measured by a conventional concentration method, can be measured.

### Examples

Hereinbelow, the present invention will be described in more detail with reference to Examples, but is not limited to these Examples.

### Example 1

### Assay of Nasal Mucus Collected by Cotton Swab

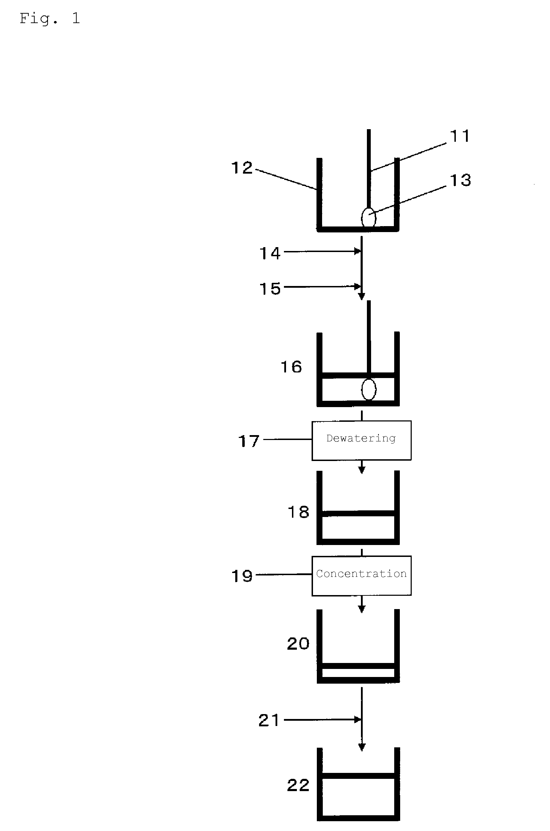

This example will be described with reference to Fig. 1.

### Sample Preparation Step

Nasal mucus was collected from 5 healthy subjects a to e by applying a method for collecting a sample for a rapid test kit for influenza with the use of cotton swabs (11) (y-sterilized aluminum shaft cotton swabs) manufactured and marketed by Eiken Chemical Co., Ltd. Each of the cotton swabs (11) used to collect nasal mucus as a sample (13) was placed in a test tube (12).

### Concentration Step

Then, 500 µL of 80% formic acid (14) and 50 µL of 10 µM SAC (15) were added to the test tube (12), in which the cotton swab (11) containing the collected sample (13) was placed, to extract and solubilize the sample (13) contained in the cotton swab to obtain a sample (16). Further, the cotton swab (11) was fixed to the test tube (12) in a state where a collection tip of the cotton swab (11) was pulled out of the solution in the test tube (12), and the test tube (12) was centrifuged by a centrifugal machine at 2000 rpm for 1 minute to subject the cotton swab (11) to dewatering (17). Then, the cotton swab (11) was taken out of the test tube (12) to obtain a sample (18). Then, the sample (18) was concentrated by vacuum concentration (19) to obtain a sample (20) having a volume of 40 µL.

### Neutralization Step

Then, 960 µL of 1M Tris buffer solution (non-adjusted pH) (21) was added to the sample (20) to neutralize formic acid to obtain a measurement sample (22) having a volume of 1 mL.

### Measurement Step

Measurement was performed using β amyloid ELISA kits (manufactured by Wako Pure Chemical Industries, Human/Rat β Amyloid42 ELISA Kit wako High-Sensitive; product number 292-64501 and Human/Rat β Amyloid40 ELISA Kit wako II; product number; 294-64701). Prior to measurement, solutions for preparing standard curves were prepared using Aβ42 and Aβ40 standard solutions or a stock solution prepared in Example 4. The concentrations of Aβ42 were adjusted to 0.05, 0.1, 0.2, 0.25, 1, 2, 2.5, 5, 10, and 20 pM and the concentrations of Aβ40 were adjusted to 0.25, 0.5, 1, 1.25, 2.5, 5, 10, 12.5, 25, 50, and 100 pM. After the solutions were prepared, 100 µL of each of the solutions for preparing standard curves and the measurement samples was added to a microtiter plate of each of the assay kits.

Then, measurement was performed according to the procedure of each of the assay kits. After the completion of measurement, the concentrations of Aβ42 and Aβ40 in each of the measurement samples were calculated using the standard curves.

### Measurement Results

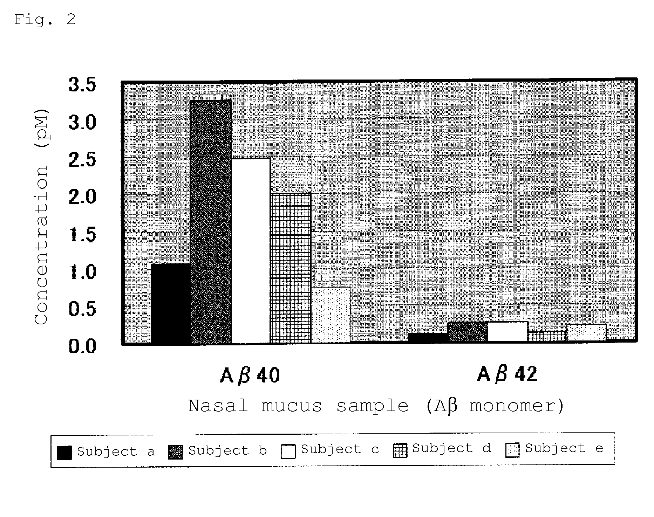

The measurement results are shown in Fig. 2. As shown in Fig. 2, the concentrations of Aβ40 and Aβ42 in the nasal mucus samples treated by the method of this example were 0.75 to 3.26 pM and 0.12 to 0.27 pM, respectively. As described above, the amount of Aβ contained in nasal mucus, which could not be measured by a conventional method, was able to be successfully measured by vacuum-concentrating a nasal mucus sample after adding formic acid.

### Example 2

### Assay of Mouse Brain Homogenate

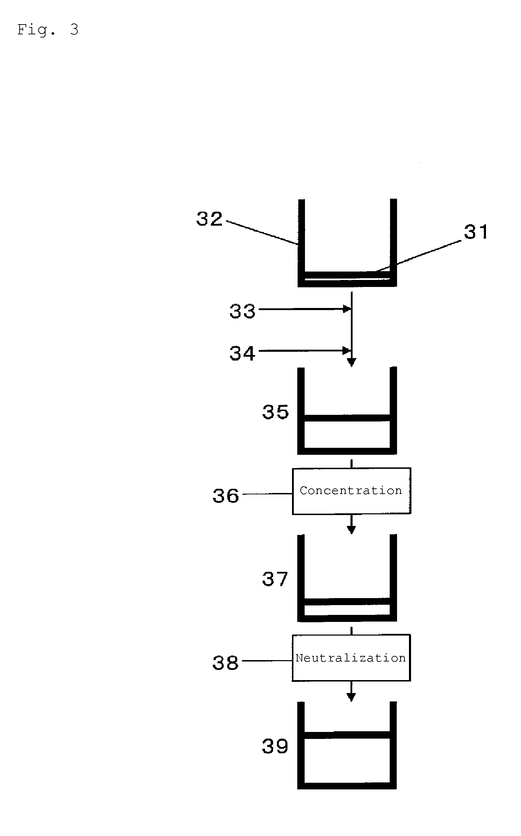

This example will be described with reference to Fig. 3.

### Sample Preparation Step

A brain slice of a mouse genetically modified to express a large amount of Aβ (hereinafter, referred to as an "APP mouse") was homogenized in 10 mM phosphate buffer (PB) to obtain a brain homogenate, and a supernatant of the brain homogenate was collected. In this example, the supernatant was used as a sample (31), and 10 µL of the sample (31) was placed in a test tube (32).

### Concentration Step

Then, 500 µL of 80% formic acid (33) and 50 µL of 10 µM SAC (34) were added to the sample (31) placed in the test tube (32) for solubilization to obtain a sample (35) having a volume of 560 µL. Then, the sample (35) was concentrated by vacuum concentration (36) to obtain a sample (37) having a volume of 40 µL.

### Neutralization Step

Then, 960 µL of 1M Tris buffer solution (non-adjusted pH) (38) was added to the sample (37) to neutralize formic acid to obtain a measurement sample (39) having a volume of 1 mL.

### Preparation of Non-Treated Sample

Ten microliters of the brain sample prepared in the sample preparation step was placed in a test tube, and then 990 µL of 10 mM phosphate buffer (PB) was added to the test tube to dilute the brain sample 100-fold. This dilution factor was the same as that of the brain sample subjected to the concentration step and the neutralization step. The thus obtained sample was defined as a non-treated sample for comparison with the treated sample of this example.

### Measurement Step

Measurement was performed using β amyloid ELISA kits (manufactured by Wako Pure Chemical Industries, Human/Rat β Amyloid42 ELISA Kit wako High-Sensitive; product number 292-64501 and Human/Rat β Amyloid40 ELISA Kit wako II; product number; 294-64701). Prior to measurement, solutions for preparing standard curves were prepared using Aβ42 and Aβ40 standard solutions or a stock solution prepared in Example 4. The concentrations of Aβ42 were adjusted to 0.1, 0.2, 0.25, 1, 2, 2.5, 5, 10, and 20 pM and the concentrations of Aβ40 were adjusted to 1, 1.25, 2.5, 5, 10, 12.5, 25, 50, and 100 pM. After the solutions were prepared, 100 µL of each of the solutions for preparing standard curves and the measurement samples was added to a microtiter plate of each of the assay kits.

Then, measurement was performed according to the procedure of each of the assay kits. After the completion of measurement, the concentrations of Aβ42 and Aβ40 in each of the measurement samples were calculated using the standard curves.

### Measurement Results

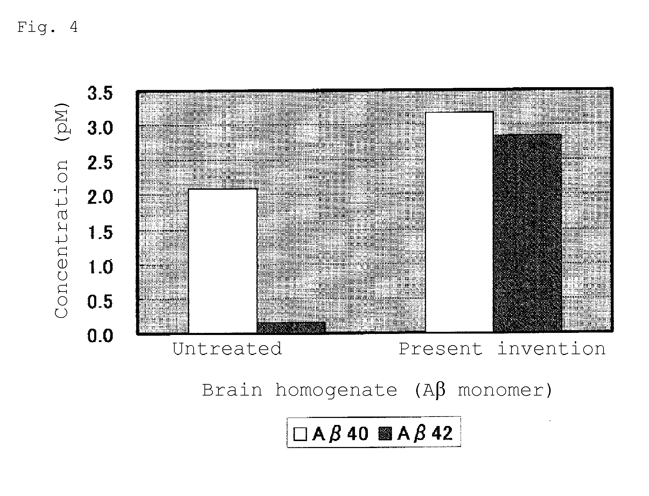

The measurement results are shown in Fig. 4. As shown in Fig. 4, the concentrations of Aβ40 and Aβ42 in the brain sample treated by the method of this example were higher than those in the non-treated sample. Particularly, the concentration of Aβ42 in the treated sample was about 18 times higher than that in the non-treated sample, from which it has been found that the brain sample contained a large amount of aggregated Aβ42. From the result, it has been found that aggregated Aβ contained in a sample, which cannot be measured by a conventional method, can be measured by vacuum-concentrating the sample after adding formic acid to convert the aggregated Aβ into Aβ monomers, and therefore very highly-sensitive measurement of Aβ can be achieved.

### Example 3

### Presence or Absence of Concentration Step after Solubilization Step

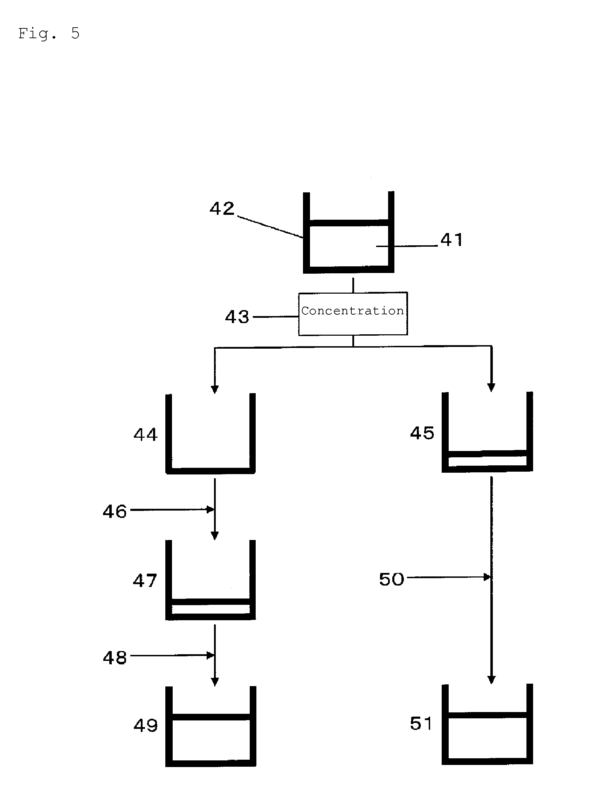

This example will be described with reference to Fig. 5.

It is to be noted that in Example 3, a dilute solution of Aβ in phosphate buffer was prepared as a simulated irrigation solution obtained by nasal irrigation, and the dilute solution was used as a sample. The concentrations of Aβ40 and Aβ42 in the sample were set to 50 pM and 10 pM, respectively. The above concentrations were estimated values obtained in the following way. The concentrations of Aβ in nasal irrigation solutions collected from APP mice were measured, and were reflected in humans by the ratio of body weight.

### Sample Preparation Step

A DMSO solution of Aβ42 (concentration: 20 µM) and a DMSO solution of Aβ40 (concentration: 100 µM) were prepared using commercially-available solid Aβ42 (manufactured by Peptide Institute Inc.; product number: 4349-V), commercially-available solid Aβ40 (manufactured by Peptide Institute Inc.; product number: 4307-V), and dimethylsulfoxide (DMSO). These solutions were mixed in equal proportions to obtain a mixture of Aβ42 and Aβ40.

### Concentration Step

Then, the mixture was diluted with 70% formic acid for solubilization so that final concentrations of Aβ42 and Aβ40 in 70% formic acid solution were 10 pM and 50 pM, respectively. The thus obtained solution was used as a sample (41). One milliliter of the sample (41) was placed in a test tube (42) and concentrated by vacuum concentration (43) so as to be completely dried and solidified to obtain a sample (44). On the other hand, 1 mL of the sample (41) was placed in another test tube (42) and concentrated by vacuum concentration (43) without being completely dried and solidified so that 50 µL of liquid remained. The remaining liquid was used as a sample (45). Then, 40 µL of formic acid (46) was added to the completely dried and solidified sample (44) to obtain a sample (47).

### Neutralization Step

Then, 960 µL of 1M Tris buffer (48) was added to the sample (47) for neutralization to obtain 1000 µL of a measurement sample (49). On the other hand, 950 µL of 1M Tris buffer (50) was directly added to the sample (45), which had not been completely dried and solidified, for neutralization to obtain 1000 µL of a measurement sample (51).

### Measurement Step

Measurement was performed using β amyloid ELISA kits (manufactured by Wako Pure Chemical Industries, Human/Rat β Amyloid42 ELISA Kit wako High-Sensitive; product number 292-64501 and Human/Rat β Amyloid40 ELISA Kit wako II; product number; 294-64701). Prior to measurement, solutions for preparing standard curves were prepared using Aβ42 and Aβ40 standard solutions or a stock solution prepared in Example 4. The concentrations of Aβ42 were adjusted to 0.3125, 0.625, 1.25, 2.5, 5, 10 and 20 pM and the concentrations of Aβ40 were adjusted to 1.5625, 3.125. 6.25, 12.5, 25, 50, and 100 pM. After the solutions were prepared, 1 mL of each of the solutions for preparing standard curves and the test samples was added to microtiter plates of the assay kits.

Then, measurement was performed according to the procedure of each of the assay kits. After the completion of measurement, the concentrations of Aβ42 and Aβ40 in each of the measurement samples were calculated using the standard curves and corrected for volume to evaluate the recovery rates of Aβ42 and Aβ40 contained in each of the samples.

### Measurement Results

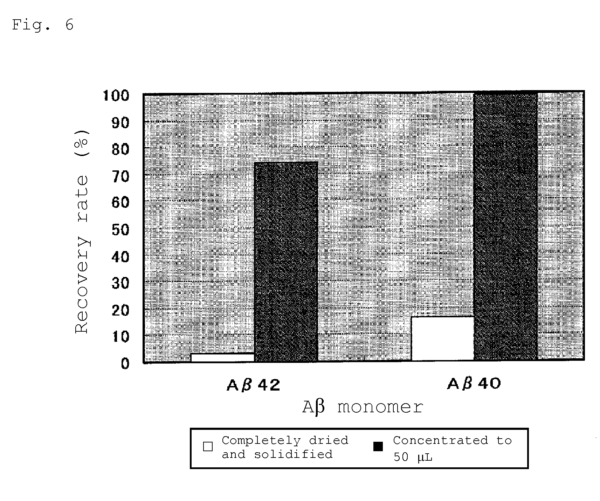

The measurement results are shown in Fig. 6. As can be seen from Fig. 6, in the case of the sample not dried and solidified in the concentration step performed after adding a solubilizer, the recovery rates of Aβ42 and Aβ40 were as very high as 80% and 100%, respectively. On the other hand, in the case of the sample dried and solidified in the concentration step performed after adding a solubilizer, the recovery rates of Aβ42 and Aβ40 were as very low as less than 10% and less than 20%, respectively. From the results, it has been found that the recovery rate of Aβ, that is, measurement sensitivity, is significantly improved by concentrating a sample without drying and solidifying it in the concentration step performed after adding a solubilizer.

### Example 4

### Study of Additive for Use in Sample Treatment Vessel Preparation Step

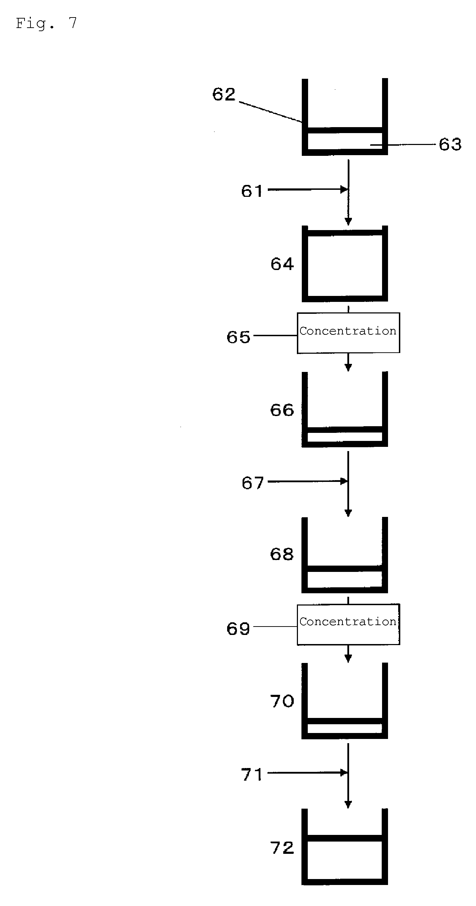

This example will be described with reference to Fig. 7.

### Preparation of Sample (61)

Preparation of a phosphate buffer solution of Aβ monomers such as Aβ42 and Aβ40 involves loss of Aβ monomers due to their aggregative properties and adsorptive properties. Therefore, a phosphate buffer solution of Aβ monomers was carefully prepared based on a serial dilution method. More specifically, a DMSO solution of Aβ42 (concentration: 20 µM) and a DMSO solution of Aβ40 (concentration: 100 µM) were prepared using commercially-available solid Aβ42 (manufactured by Peptide Institute Inc.; product number: 4349-V), commercially-available solid Aβ40 (manufactured by Peptide Institute Inc.; product number: 4307-V), and dimethylsulfoxide (DMSO). These solutions were mixed in equal proportions to obtain a mixture of Aβ42 and Aβ40. Then, the mixture was diluted with 0.5% bovine serum albumin (BSA) and 0.1% Tween20 phosphate buffered saline (pH 7.4, PBS) until the concentration of Aβ42 became 5 nM and the concentration of Aβ40 became 25 nM. In the diluted mixture, both Aβ42 and Aβ40 can be present stably as Aβ monomers. Therefore, the diluted mixture was used as a stock solution in this example.

Then, the stock solution was diluted with phosphate buffer (PB) to a desired concentration by a serial dilution method. In this example, the final concentrations of Aβ42 and Aβ40 in phosphate buffer solution were set to 5 pM and 25 pM, respectively. Further, in the above-described process of serial dilution, bovine serum albumin (BSA) was added as a blocking agent to achieve a final concentration of 0.9%. Then, the prepared solution was allowed to stand for 24 hours or longer to obtain, as a sample (61), a mixture solution of soluble Aβ oligomers, insoluble Aβ polymers, and Aβ monomers.

The thus prepared sample was a 5 pM Aβ42·25 pM Aβ40·0.9% BSA phosphate buffer solution (containing a trace amount of DMSO).

### Sample Treatment Vessel Preparation Step

Thirteen test tubes (62) were prepared, and 250 µL of an additive (63) was added to each of the test tubes. Thirteen kinds of additives (A) to (M) listed below were prepared as the additives (63). One kind of additive was added per test tube.
(A) 0.9% BSA·80% formic acid
(B) 10 nM curcumin·0.9% BSA·80% formic acid
(C) 9% Triton x 100·0.9% BSA·80% formic acid
(D) 10 µM SAC·0.9% BSA·80% formic acid
(E) 20% DMSO·0.9% BSA·80% formic acid
(F) 0.9% BSA·0.1% Tween20·PBS
(G) 10 nM curcumin·0.9% BSA·0.1% Tween20·PBS
(H) 9% Triton x 100·0.9% BSA·0.1% Tween20·PBS(I) 10 µM SAC·0.9% BSA·0.1% Tween20·PBS
(J) 50% DMSO·0.9% BSA·0.1% Tween20·PBS
(K) PB
(L) 28%NH₃
(M) 5.6% NH₃·0.9% BSA·0.1% Tween20·PBS

### Sample Preparation Step

Then, 2 mL of the sample (61) was added to each of the test tubes to obtain 2250 µL of a sample (64).

### First Concentration Step

The sample (64) was subjected to concentration (65) by vacuum concentration to obtain a sample (66) having a volume of 50 µL.

### Second Concentration Step

Three hundred microliters of 80% formic acid (67) was added to the sample (66) for solubilization to obtain a sample (68) having a volume of 350 µL. Then, the sample (67) was concentrated by vacuum concentration (69) to obtain a sample (70) having a volume of 40 µL.

### Neutralization Step

Then, 960 µL of 1M Tris buffer solution (non-adjusted pH) (71) was added to the sample (70) to neutralize formic acid to obtain a measurement sample (72) having a volume of 1 mL.

### Measurement Step

Measurement was performed using β amyloid ELISA kits (manufactured by Wako Pure Chemical Industries, Human/Rat β Amyloid42 ELISA Kit wako High-Sensitive; product number 292-64501 and Human/Rat β Amyloid40 ELISA Kit wako II; product number; 294-64701). Prior to measurement, solutions for preparing standard curves were prepared using Aβ42 and Aβ40 standard solutions or the stock solution prepared above. The concentrations of Aβ42 were adjusted to 0.3125, 0.625, 1.25, 2.5, 5, 10 and 20 pM and the concentrations of Aβ40 were adjusted to 1.5625, 3.125. 6.25, 12.5, 25, 50, and 100 pM. After the solutions were prepared, each of the solutions for preparing standard curves and the measurement samples (72) was added to microtiter plates of the assay kits.

Then, measurement was performed according to the procedure of each of the assay kits. After the completion of measurement, the concentrations of Aβ42 and Aβ40 in each of the measurement samples (72) were calculated using the standard curves and corrected for volume to evaluate the recovery rates of Aβ42 and Aβ40 contained in each of the samples.

### Measurement Results

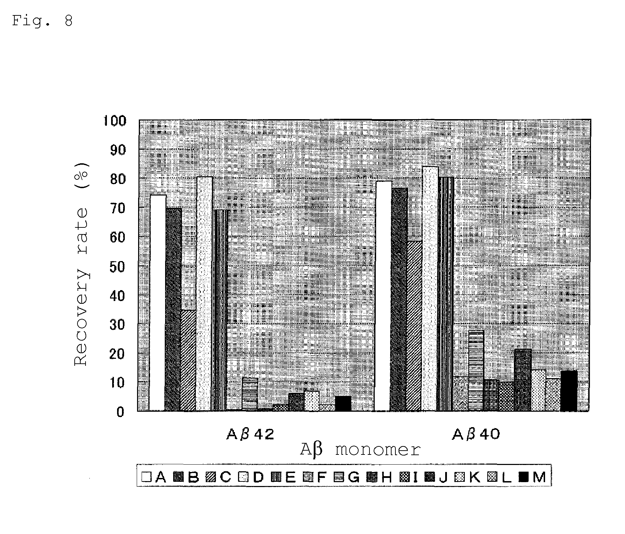

The measurement results are shown in Fig. 8. As shown in Fig. 8, the recovery rates of both Aβ42 and Aβ40 were much higher in the cases of samples (A) to (E) obtained by previously adding formic acid to the sample treatment vessel than in the cases of samples (F) to (M) obtained by adding no formic acid. Particularly, in the case of sample (D) obtained by using both formic acid and SAC, the recovery rates of Aβ42 and Aβ40 were highest. From the results, it has been found that, even when the pre-concentration step (first concentration step) is performed prior to the concentration step (second concentration step), the recovery rate of Aβ, that is, measurement sensitivity is significantly improved by previously adding an additive typified by formic acid to a sample treatment vessel.

### Reference Example

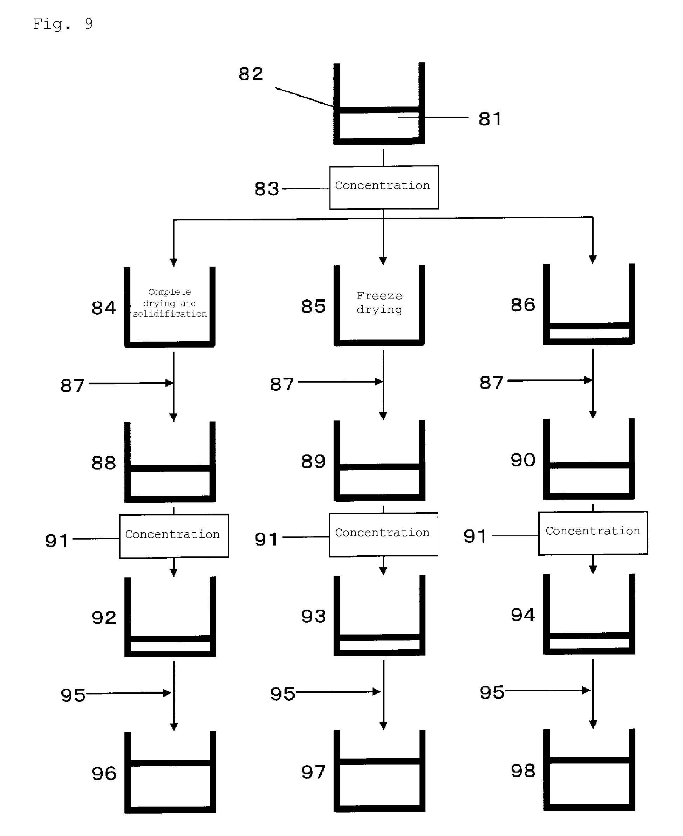

The necessity of an additive when the first concentration step is performed was examined by performing the first concentration step without using an additive used in Example 4. Reference Example will be described with reference to Fig. 9.

### Preparation of Sample (81)

A DMSO solution of Aβ42 (concentration: 20 µM) and a DMSO solution of Aβ40 (concentration: 100 µM) were prepared using commercially-available solid Aβ42 (manufactured by Peptide Institute Inc.; product number: 4349-V), commercially-available solid Aβ40 (manufactured by Peptide Institute Inc.; product number: 4307-V), and dimethylsulfoxide (DMSO). These solutions were mixed in equal proportions to obtain a mixture of Aβ42 and Aβ40. Then, the mixture was diluted with 0.5% bovine serum albumin (BSA) and 0.1% Tween20 phosphate buffered saline (pH 7.4, PBS) until the concentration of Aβ42 became 5 nM and the concentration of Aβ40 became 25 nM. In this diluted mixture, both Aβ42 and Aβ40 can be present stably as Aβ monomers. Therefore, the diluted mixture was used as a stock solution in Reference Example.

Then, the stock solution was diluted with phosphate buffer (PB) to a desired concentration by a serial dilution method. In Reference Example, the final concentrations of Aβ42 and Aβ40 in phosphate buffer solution were set to 20 pM and 100 pM, respectively. Further, in the above-described process of serial dilution, bovine serum albumin (BSA) was added as a blocking agent to achieve a final concentration of 0.9%. Then, the prepared solution was allowed to stand for 24 hours or longer to obtain, as a sample (81), a mixture solution of soluble Aβ oligomers, insoluble Aβ polymers, and Aβ monomers.

The thus prepared sample was a 20 pM Aβ42·100 pM Aβ40·0.9% BSA phosphate buffer solution (containing a trace amount of DMSO).

### First Concentration Step

Five hundred microliters of the sample (81) was added to a test tube (82), and then the sample (81) was subjected to concentration (83) by vacuum concentration. Here, the concentration (83) was performed by freeze drying or vacuum concentration to prepare a sample (84) completely dried and solidified by vacuum concentration, a freeze-dried sample (85), and a sample (86) vacuum-concentrated to a volume of 50 µL without being dried and solidified.

### Second Concentration Step

Seventy percent formic acid (87) was added to each of the samples for solubilization to obtain samples (88), (89), and (90) each having a volume of 500 µL.

Then, the samples (88), (89), and (90) were subjected to concentration (91) by vacuum concentration without being completely dried and solidified to obtain samples (92), (93), and (94) each having a volume of 40 µL.

### Neutralization Step

Then, 960 µL of 1M Tris buffer solution (non-adjusted pH) (95) was added to each of the samples (92), (93), and (94) to neutralize formic acid to obtain measurement samples (96), (97), and (98) each having a volume of 1 mL.

### Measurement Step

Measurement was performed using β amyloid ELISA kits (manufactured by Wako Pure Chemical Industries, Human/Rat β Amyloid42 ELISA Kit wako High-Sensitive; product number 292-64501 and Human/Rat β Amyloid40 ELISA Kit wako II; product number; 294-64701). Prior to measurement, solutions for preparing standard curves were prepared using Aβ42 and Aβ40 standard solutions or the stock solution prepared above. The concentrations of Aβ42 were adjusted to 0.3125, 0.625, 1.25, 2.5, 5, 10 and 20 pM and the concentrations of Aβ40 were adjusted to 1.5625, 3.125. 6.25, 12.5, 25, 50, and 100 pM. After the solutions were prepared, 1 mL of each of the solutions for preparing standard curves and the measurement samples was added to microtiter plates of the assay kits.

Then, measurement was performed according to the procedure of each of the assay kits. After the completion of measurement, the concentrations of Aβ42 and Aβ40 in each of the measurement samples were calculated using the standard curves and corrected for volume to evaluate the recovery rates of Aβ42 and Aβ40 contained in each of the samples.

### Measurement Results

The measurement results are shown in Fig. 10. As can be seen from Fig. 10, in any of the cases of the samples, the recovery rate of Aβ40 was as low as about 24%. When the first concentration step was performed prior to addition of a solubilizer to concentrate the sample (81), the recovery rate of Aβ was low and there was no significant difference in the recovery rate between when the sample was completely dried and solidified and when the sample was not completely dried and solidified. As described above with reference to Example 4, it has been found that when the first concentration step is performed prior to addition of a solubilizer, previously adding an additive such as formic acid to a sample treatment vessel is more important than avoiding complete drying and solidification of a sample in the first concentration step to increase the recovery rate of Aβ, that is, measurement sensitivity.

### INDUSTRIAL APPLICABILITY

The Aβ measurement method according to the present invention makes it possible to keep Aβ in a solubilization state even when a sample is concentrated and therefore to quantitatively measure Aβ contained in a solution in low concentration accurately. Therefore, the method according to the present invention makes it possible to measure a trace amount of Aβ present in, for example, an irrigation solution obtained by nasal irrigation, and can be used in early in-vitro diagnosis of Alzheimer's disease.

### Description of Reference Numerals

11 cotton swab
12 test tube
13 sample
14 addition of formic acid
15 addition of SAC
16 formic acid- and SAC-containing sample
17 dewatering of cotton swab
18 formic acid- and SAC-containing sample obtained after removal of cotton swab
19 concentration
20 sample concentrated to volume of 40 µL
21 addition of 1M Tris buffer solution
22 measurement sample
31 sample
32 test tube
33 addition of formic acid
34 addition of SAC
35 formic acid- and SAC-containing sample
36 concentration
37 sample concentrated to volume of 40 µL
38 addition of 1M Tris buffer solution
39 measurement sample
41 sample
42 test tube
43 concentration
44 sample completely dried and solidified
45 sample not completely dried and solidified
46 addition of formic acid
47 formic acid-containing sample
48, 50 addition of 1M Tris buffer solution
49 measurement sample
51 measurement sample
61 sample
62 test tube
63 additive
64 additive-containing sample
65, 69 concentration
66 concentrated sample not completely dried and solidified
67 addition of formic acid
68 formic acid-containing sample
70 sample concentrated by second concentration without being completely dried and solidified
71 addition of 1M Tris buffer solution
72 measurement sample
81 sample
82 test tube
83, 91 concentration
84 sample completely dried and solidified by first concentration
85 freeze-dried sample
86 sample concentrated by first concentration without being completely dried and solidified
87 addition of formic acid
88, 88, 89 formic acid-containing sample
92, 93, 94 sample concentrated by second concentration without being completely dried and solidified
95 addition of 1M Tris buffer solution
96, 97, 98 measurement sample
101 sample preparation step
102 concentration step
103 neutralization step
104 measurement step
111 sample treatment vessel preparation step
112 sample preparation step
113 first concentration step
114 second concentration step
115 neutralization step
116 measurement step

## Claims

1. An amyloid β measurement method comprising: a sample preparation step in which a sample possibly containing amyloid β is placed in a sample treatment vessel; a concentration step in which a solubilizer that solubilizes amyloid β is added to the sample in the sample treatment vessel and an amount of solvent contained in the sample is reduced by a concentration operation; a neutralization step in which the solubilizer in a treated sample solution obtained in the concentration step is neutralized; and a measurement step in which amyloid β possibly contained in a neutralized treated sample solution is quantitatively measured based on an antigen-antibody reaction.

2. The amyloid β measurement method according to claim 1, further comprising, prior to the sample preparation step, a sample treatment vessel preparation step in which an additive to be attached to amyloid β is placed in a sample treatment vessel.

3. An amyloid β measurement method comprising: a sample treatment vessel preparation step in which an additive to be attached to amyloid β is placed in a sample treatment vessel; a sample preparation step in which a sample possibly containing amyloid β is placed in the sample treatment vessel; a first concentration step in which the sample in the sample treatment vessel is concentrated; a second concentration step in which a solubilizer that solubilizes amyloid β is added to the sample concentrated in the first concentration step and an amount of solvent contained in the sample is reduced by a concentration operation; a neutralization step in which the solubilizer in a treated sample solution obtained in the second concentration step is neutralized; and a measurement step in which amyloid β possibly contained in a neutralized treated sample solution is quantitatively measured based on an antigen-antibody reaction.

4. The amyloid β measurement method according to claim 1 or 3, wherein the solubilizer is formic acid.

5. The amyloid β measurement method according to claim 2 or 3, wherein the additive contains formic acid and S-allyl-L-cysteine.

6. The amyloid β measurement method according to claim 1 or 3, wherein the sample treatment vessel contains a blocking agent.

7. The amyloid β measurement method according to claim 6, wherein the blocking agent contains bovine serum albumin.

8. The amyloid β measurement method according to claim 1 or 3, wherein the sample treatment vessel has an inner wall surface that inhibits adsorption of amyloid β.

9. The amyloid β measurement method according to claim 3, wherein in the first concentration step, concentration is performed without insolubilizing amyloid β.

10. The amyloid β measurement method according to claim 1 or 3, wherein the sample is a body fluid.

11. The amyloid β measurement method according to claim 1 or 3, wherein the sample is an irrigation solution obtained by irrigation of living tissue.

12. The amyloid β measurement method according to claim 11, wherein the living tissue is nasal mucosa.
